# EUROPÄISCHE PATENTSCHRIFT

(11) **EP 4 076 605 B1**
(45) Veröffentlichungstag und Bekanntmachung des Hinweises auf die Patenterteilung: **12.03.2025**
(21) Anmeldenummer: 19839323.3
(22) Anmeldetag: 18.12.2019
(51) Int. Cl.: A61M 15/00

(54) **INHALATOR**
INHALER
INHALATEUR

(43) Veröffentlichungstag der Anmeldung: 26.10.2022
(73) Patentinhaber: Mezger, Markus, 71691 Freiberg am Neckar (DE)
(72) Erfinder: Mezger, Markus, 71691 Freiberg am Neckar (DE)
(74) Vertreter: Neu, Felix
(86) Internationale Anmeldenummer: PCT/DE2019/101106
(87) Internationale Veröffentlichungsnummer: WO 2021/121448

(56) Entgegenhaltungen:
- WO-A1-2011/004287
- WO-A2-2010/036355
- DE-U1- 202012 003 962
- US-A1- 2002 170 560
- US-A1- 2010 078 022

## Beschreibung

Die vorliegende Erfindung betrifft einen Inhalator zur Verabreichung pulverförmiger Trockensubstanzen.

### Stand der Technik

Vorrichtungen zur Verabreichung von pulverförmigen Trockensubstanzen, z. B. pharmazeutischen Präparaten, durch Inhalation bestehen in bekannten Ausführungen aus einem in der Regel etwa handtellergroßen Gerät, in dessen Inneren sich die aktiven Substanzen, beispielsweise in einem aufgewickelten Blisterstreifen aus tiefgezogener Aluminiumfolie befinden. Dieser Streifen wird durch relative Bewegung verschiedener Gehäuseteile, beispielsweise in Form von Schutzkappen oder durch separate Schwenkhebel für jede Anwendung schrittweise weiter bewegt, wobei die jeweiligen, die Substanz enthaltenden Blisterkavitäten geöffnet und dadurch die aktiven Substanzen zur Inhalation freigegeben werden.

Auch sind Inhalatoren bekannt, die zur Verabreichung von zwei verschiedenen Trocken-Substanzen von beispielsweise pharmazeutischen Präparaten zwei aufgewickelte Blisterstreifen verwenden. Solche Inhalatoren werden beispielsweise in der WO 03/0617 43 A1, in der WO 2005/079727 A2 und in der WO 2005/014089 A1 beschrieben.

In der Regel sind auf solchen Blisterstreifen 30 oder 60 Einzeldosen untergebracht, um den Monatsbedarf eines Patienten bei einmaliger oder zweimaliger Inhalationen pro Tag abdecken zu können. Bei einem Doppelblisterstreifen-Inhalator werden zeitgleich zwei verschiedene aktive Substanzen dosiert.

Die Öffnung der Blisterkavitäten erfolgt beispielsweise durch schrittweises Abziehen einer die Kavitäten abdeckenden Folie, wobei sowohl die Deckfolie als auch der entleerte Blisterstreifen im Inhalator aufgewickelt oder anderweitig verwahrt werden müssen. Dies setzt eine meist recht aufwendige Mechanik voraus und ist wegen des biegeschlaffen Blisterstreifens nur mit hohem Aufwand automatisch zu montieren.

Bei Doppelblisterstreifen-Inhalatoren ist außerdem eine aufwendige Mechanik mit sehr vielen Teilen notwendig um die aktive Trockensubstanz dem Patienten verabreichen zu können. Ein unter der Bezeichnung Ellipta^{®} vertriebener Inhalator besteht beispielsweise aus 28 Teilen.

Des weiteren sind Inhalatoren bekannt, bei denen an Stelle des Blisterstreifens eine in der Regel annähernd kreisrunde Kunststoffscheibe mit einer Anzahl von tiefgezogenen Kavitäten oder Bohrungen zur Aufnahme der pulverförmigen Substanzen verwendet wird, wobei der Verschluss der Kavitäten nach deren Befüllung mittels einer aufgesiegelten Deckfolie erfolgt. Die Kunststoffscheibe wird bei jeder Benutzung des Gerätes um einen Schritt weiter getaktet, bis alle Kavitäten geöffnet und durch Inhalation des Pulvers entleert sind. Zur Inhalation werden bei diesen Geräten die Kavitäten durch ein Entnahmeelement mittels einer eingedrückten Spitze geöffnet und somit die aktive Substanz zur Inhalation freigegeben. Ein solcher Inhalator ist aus der WO 2013/068579 A1 bekannt.

Derartige Inhalatoren enthalten allerdings nur eine Kunststoffscheibe, so dass sie nur die Verabreichung einer Substanz ermöglichen.

Aus der WO 2011/004287 A1 ist ein Inhalator mit zwei Trägerscheiben bekannt. Jeder Trägerscheibe ist jeweils ein bezüglich der Richtung einer gemeinsamen Drehachse der Trägerscheiben außenliegender Luftkanal zugeordnet.

Es ist eine Aufgabe der vorliegenden Erfindung, einen Inhalator bereitzustellen, der die gleichzeitige Verabreichung von zwei pulverförmigen Trockensubstanzen ermöglicht und einfacher zu fertigen ist als ein herkömmlicher Doppelblisterstreifen-Inhalator. Insbesondere sollte der Inhalator weniger Teile aufweisen als ein herkömmlicher Doppelblisterstreifen-Inhalator.

### Offenbarung der Erfindung

Diese Aufgabe wird durch einen Inhalator zur Verabreichung pulverförmiger Trockensubstanzen gelöst, welcher zwei Substanzträgerscheiben aufweist.

Jede Substanzträgerscheibe weist jeweils mit einer pulverförmigen Trockensubstanz befüllte Kavitäten auf, vorzugsweise jeweils mindestens 30 Kavitäten. Bei den pulverförmigen Trockensubstanzen kann es sich insbesondere um Substanzen mit einer Partikelgröße im Bereich von 2 µm bis 100 µm handeln. Die Kavitäten der ersten Substanzträgerscheibe sind insbesondere mit einer anderen pulverförmigen Trockensubstanz gefüllt, als die Kavitäten der zweiten Substanzträgerscheibe. Die Substanzträgerscheiben sind so um eine gemeinsame Drehachse drehbar angeordnet, dass sich die Kavitäten jeweils gegenüberliegen. Der Inhalator weist weiterhin einen Luftkanal auf. Der Luftkanal weist eine Anstechdüse auf, die zwischen den Substanzträgerscheiben angeordnet ist. Diese Anordnung des Luftkanals, durch welchen ein Gemisch aus Luft und einer pulverförmigen Trockensubstanz zu einem Mundstück des Inhalators geleitet werden kann, ermöglicht eine ähnliche räumliche Distanz und eine ähnliche geometrische Lage einer Entnahmeposition aus einer anzustechenden Kavität zum Mundstück, wie diese in einem Inhalator mit einer Substanzträgerscheibe üblicherweise vorgesehen ist. Dadurch kann dasselbe aerodynamische Verhalten der Trockensubstanzpartikel in der Luftströmung erreicht werden, sodass ähnliche Inhalationsparameter wie in bekannten Inhalatoren mit nur einer Substanzträgerscheibe erreicht werden können.

Indem die pulverförmige Trockensubstanz nicht in Blisterstreifen, sondern in Substanzträgerscheiben bevorratet wird, ist es möglich, den erfindungsgemäßen Inhalator in einfacher Weise und unter Verwendung von wenigen Teilen herzustellen. Da Teile von Inhalatoren üblicherweise in einem Spritzgussverfahren aus thermoplastischen Kunststoffen hergestellt werden und für jedes zu fertigende Teil ein separates Spritzgusswerkzeug angefertigt werden muss, führt dies zu einer erheblichen Kostenersparnis bei der Herstellung des erfindungsgemäßen Inhalators.

Durch Drehung der Substanzträgerscheiben können die mit der pulverförmigen Trockensubstanz befüllten Kavitäten nacheinander der zwischen den Substanzträgerscheiben angeordneten Anstechdüse zugewandt werden, um die Trockensubstanzen in den Luftkanal zu transportieren. Um diese Drehbewegung zu realisieren, ist es bevorzugt, dass jede Substanzträgerscheibe an ihrer Mantelfläche jeweils eine Nutkurve aufweist, in welche jeweils ein Kurvenführungszapfen eingreift. Durch eine Bewegung des jeweiligen Kurvenführungszapfens kann dann eine Drehbewegung einer Substanzträgerscheibe ausgelöst werden.

Vorzugsweise sind die Nutkurven und die Kurvenführungszapfen eingerichtet, um nicht nur eine Drehbewegung der Substanzträgerscheiben, sondern gleichzeitig eine lineare Verschiebung der Substanzträgerscheiben entlang der Drehachse zu bewirken. Dies kann durch eine geeignete Form der Nutkurven realisiert werden. Eine Bewegung der Kurvenführungszapfen ermöglicht es dann, die Substanzträgerscheiben nicht nur zur jeweils nächsten Kavität weiterzudrehen, sondern sie gleichzeitig auch zu der Anstechdüse hin zu bewegen, um so ein Anstechen der Kavitäten durch die Anstechdüse zu ermöglichen. Es können also zwei Bewegungen gleichzeitig durchgeführt werden.

Vorzugsweise können die Substanzträgerscheiben jeweils nur in eine Richtung gedreht werden, Ein Zurückdrehen kann insbesondere durch eine geeignete Form der Nutkurven verhindert werden. Auf diese Weise wird ausgeschlossen, dass Kavitäten, die bereits entleert wurden, erneut mit der Anstechdüse zusammengeführt werden.

Weiterhin ist es bevorzugt, dass die Nutkurven eingerichtet sind, um eine Anzahl von Drehungen ihrer Substanzträgerscheiben zu begrenzen. Dies kann insbesondere dadurch realisiert werden, dass die Nutkurven jeweils eine Unterbrechung aufweisen. Wenn alle Kavitäten der Substanzträgerscheiben entleert wurden, wird auf diese Weise verhindert, dass durch ein Weiterdrehen der Substanzträgerscheiben erneut versucht wird, eine pulverförmige Trockensubstanz aus einer bereits zuvor entleerten Kavität zu entnehmen.

Es ist bevorzugt, dass der Inhalator ein Betätigungselement aufweist, welches die Kurvenführungszapfen aufweist. Das Betätigungselement ermöglicht es einem Nutzer des Inhalators, die Substanzträgerscheiben in Bewegung zu setzen und sie so zu den nächsten befüllten Kavitäten weiterzudrehen und diese Kavitäten mit der Anstechdüse anzustechen. Indem beide Kurvenführungszapfen an demselben Betätigungselement angeordnet sind, wird eine gleichzeitige Betätigung beider Substanzträgerscheiben ermöglicht.

Jeder Kurvenführungszapfen ist vorzugsweise an einem federnden Ende des Betätigungselements angeordnet. Dies ermöglicht es, dass die Kurvenführungszapfen nach einer Betätigung des Betätigungselements in eine Ausgangsposition zurückfedern.

Die Kurvenführungszapfen sind vorzugsweise so dem Betätigungselement angeordnet, dass sie bei Betätigung des Betätigungselements eine synchrone Bewegung der beiden Substanzträgerscheiben bewirken. Wenn beide Substanzträgerscheiben sich gleichzeitig in die gleiche Richtung drehen und mit der gleichen Geschwindigkeit auf die Anstechdüse zubewegt werden, wird sichergestellt, dass die Trockensubstanzen der beiden Substanzträgerscheiben in gleicher Weise im Luftkanal gemischt werden.

Die in den Substanzträgerscheiben angeordneten Kavitäten sind bevorzugt jeweils mit einer Deckfolie versiegelt. Um eine einfache Fertigung der Substanzträgerscheiben zu ermöglichen, handelt es sich hierzu bei jeder Substanzträgerscheibe vorzugsweise um eine gemeinsame Deckfolie, die gleichzeitig alle Kavitäten der Substanzträgerscheibe bedeckt. Insbesondere kann es sich bei der Deckfolie um eine Aluminiumfolie handeln. Wenn die Substanzträgerscheiben auf die Anstechdüse zubewegt werden, so durchsticht diese die Deckfolien und ermöglicht damit den Zugang zu der bis dahin luftdicht versiegelten Trockensubstanz.

Die Deckfolie weist vorzugsweise eine Dicke im Bereich von 20 µm bis 50 µm auf, um ein einfaches Durchstechen mittels der Anstechdüse zu ermöglichen.

Die Anstechdüse ist so ausgeführt, dass sie zwei Hohldüsen aufweist. Die Hohldüsen weisen jeweils eine Länge von vorzugsweise mindesten 2 mm auf. Jede der beiden Hohldüsen ist dabei einer der Substanzträgerscheiben zugewandt. Auf diese Weise kann die eine Hohldüse eine Kavität der einen Substanzträgerscheibe anstechen, während die andere Hohldüse eine Kavität der anderen Substanzträgerscheibe ansticht. Die Trockensubstanzen aus den beiden Kavitäten werden dann durch die beiden Hohldüsen transportiert, in der Anstechdüse gemischt und durch den Luftkanal weitertransportiert.

Um ein einfaches Durchstehen einer Deckfolie zu ermöglichen, sind die Hohldüsen vorzugsweise scharfkantig ausgeführt.

Weiterhin weist die Anstechdüse vorzugsweise mehrere, insbesondere vier, Anstechelemente aufweist. Jeder Substanzträgerscheibe sind jeweils eine Hohldüse und mehrere, insbesondere zwei, Anstechelemente zugewandt sind. Die Anstechelemente können weitere Öffnungen in die Deckfolie stechen oder schneiden, um so während eines Inhalationsvorgangs ein Einströmen von Luft in die Kavitäten zu ermöglichen. Durch die Verwendung mehrerer Anstechelemente pro Hohldüse kann die Luftführung so erfolgen, dass eine gleichmäßige und vollständige Entleerung der Kavitäten gewährleistet wird.

Der Luftkanal weist vorzugsweise eine Auslassdüse auf. Diese kann an dem der Auslassdüse gegenüberliegende Ende des Luftkanals angeordnet sein und mit einem Mundstück des Inhalators verbunden sein. Wenn die Anstechdüse jeweils eine Kavität beider Substanzträgerscheiben angestochen hat und auf diese Weise eine Verbindung zu den darin bevorrateten Trockensubstanzen hergestellt hat, kann ein Benutzer des Inhalators durch das Mundstück einatmen und dabei einen Luftsog durch den Luftkanal erzeugen. Durch diesen Luftsog werden die Trockensubstanzen als Pulver-Luft-Gemisch vollständig aus ihren Kavitäten herausgesaugt, in der Anstechdüse gemischt und aus der Auslassdüse in die Atemwege des Benutzers entlassen.

An der von den Kavitäten abgewandten Seite einer Substanzträgerscheibe oder beider Substanzträgerscheiben kann eine Kavitätenkennzeichnung, insbesondere in Form einer Nummerierung vorgesehen sein. Beim Drehen der Substanzträgerscheiben wird dann durch die Kavitätenkennzeichnung für einen Benutzer sichtbar angezeigt, welche Kavitäten der Anstechdüse zugewandt sind.

Die Substanzträgerscheiben können insbesondere aus einem thermoplastischen Kunststoff bestehen. Auch der Luftkanal und die Anstechdüse können insbesondere aus einen thermoplastischen Kunststoff bestehen. Besonders bevorzugt bestehen alle Teile des Inhalators jeweils aus einem thermoplastischen Kunststoff. Aus diese Weise kann auf Metallteile verzichtet werden. Die Kunststoffteile können beispielsweise in einem Spritzgussverfahren hergestellt werden.

### Kurze Beschreibung der Zeichnungen

Ein Ausführungsbeispiel der Erfindung ist in den Zeichnungen dargestellt und wird in der nachfolgenden Beschreibung näher erläutert.
Fig. 1 zeigt eine Explosionsdarstellung eines Inhalators gemäß einem Ausführungsbeispiel der Erfindung.
Fig. 2 zeigt eine Seitenansicht eines Inhalators gemäß einem Ausführungsbeispiel der Erfindung.
Fig. 3 zeigt eine isometrische Darstellung eines Betätigungselements eines Inhalators gemäß einem Ausführungsbeispiel der Erfindung.
Fig. 4 zeigt in einer isometrischen Detailansicht, wie ein Kurvenführungszapfen in eine Nutkurve einer Substanzträgerscheibe eines Inhalators gemäß einem Ausführungsbeispiel der Erfindung eingreift.
Fig. 5 zeigt in einer isometrischen Detailansicht die Anordnung einer Anstechdüse relativ zu Kavitäten in den Substanzträgerscheiben eines Inhalators gemäß einem Ausführungsbeispiel der Erfindung.
Fig. 6 zeigt eine Seitenansicht einer Substanzträgerscheibe eines Inhalators gemäß einem Ausführungsbeispiel der Erfindung.
Fig. 7 zeigt eine isometrische Schnittdarstellung einer Substanzträgerscheibe in einem Inhalator gemäß einem Ausführungsbeispiel der Erfindung, die eine Deckfolie aufweist.

### Ausführungsbeispiele der Erfindung

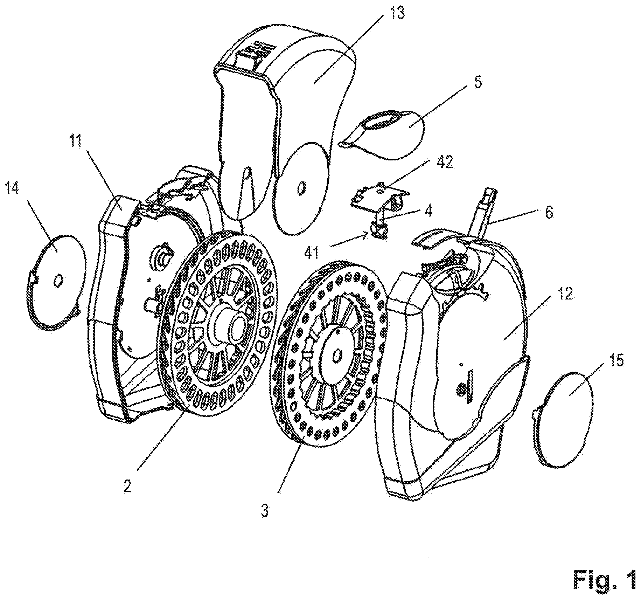

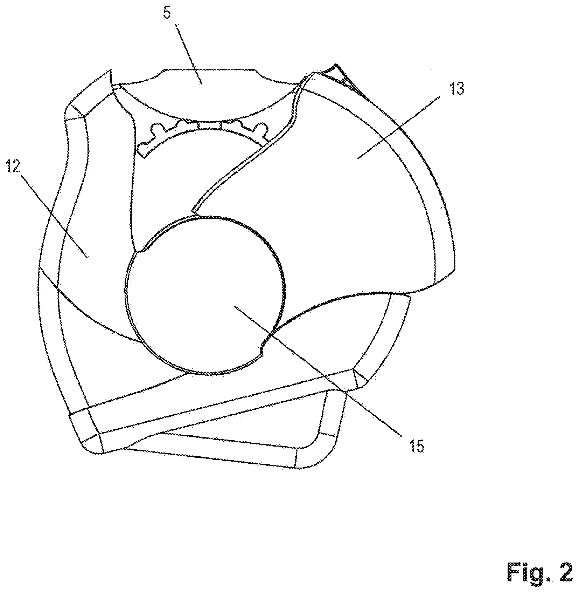

Die Fig. 1 und 2 zeigen einen Inhalator gemäß einem Ausführungsbeispiel der Erfindung, der aus lediglich zehn Teilen hergestellt werden kann. Diese können aus einem thermoplastischen Kunststoff in einem Spritzgussverfahren hergestellt werden. Das Gehäuse des Inhalators besteht aus einer ersten Gehäusehälfte 11 und einer zweiten Gehäusehälfte 12. Wenn diese zusammengesetzt werden, so wird eine Abdeckung 13 so auf die beiden Gehäusehälften 11, 12 aufgesetzt, dass sie diese umschließt. Ein erster Deckel 14 wird von der Seite der ersten Gehäusehälfte 11 auf die Abdeckung 13 aufgesetzt. Ein zweiter Deckel 15 wird von der Seite der zweiten Gehäusehälfte 12 auf die Abdeckung 13 aufgesetzt. Hierdurch wird die Abdeckung 13 auf den beiden Gehäusehälften 11, 12 fixiert.

In dem Gehäuse sind eine erste Substanzträgerscheibe 2 und eine zweite Substanzträgerscheibe 3 angeordnet. Diese sind drehbar auf einer gemeinsamen Drehachse angeordnet, um welche auch die Abdeckung 13 schwenkbar ist. Ein Luftkanal 4, der eine Anstechdüse 41 und eine Auslassdüse 42 aufweist, ist so in dem Gehäuse angeordnet, dass die Anstechdüse 41 zwischen Kavitäten der beiden Substanzträgerscheiben 2, 3 positioniert ist. Die Auslassdüse 42, die an dem der Anstechdüse 41 abgewandten Ende des Luftkanals 4 angeordnet ist, weist zu einer Öffnung im Gehäuse. Auf diese Öffnung ist ein Mundstück 5 aufgesetzt.

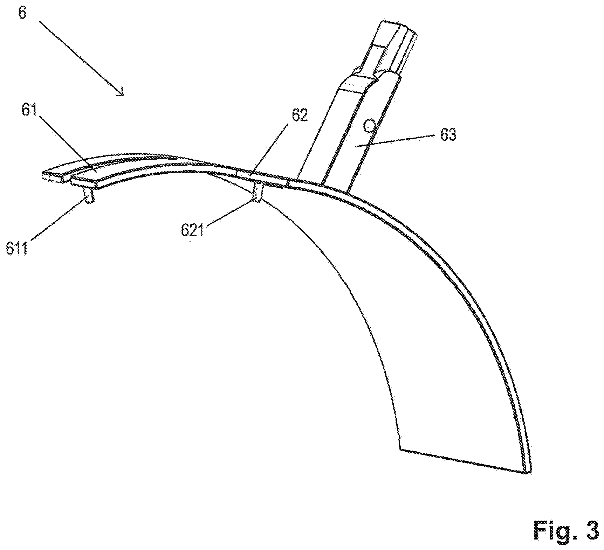

Ein Betätigungselement 6 ist so in dem Inhalator angeordnet, dass es auf den beiden Gehäusehälften 11, 12 aufliegt. Wie in Fig. 3 dargestellt ist, ist es kreisbogenförmig ausgeführt und weist zwei durch einen Spalt voneinander getrennte, parallel verlaufende federnde Enden 61, 62 auf. An jedem federnden Ende 61, 62 ist jeweils ein Kurvenführungszapfen 611, 621 so angeordnet, dass er in das Gehäuse hineinragt. Dabei greift der erste Kurvenführungszapfen 611 in eine erste Nutkurve an der Mantelfläche der ersten Substanzträgerscheibe 2 ein und der zweite Kurvenführungszapfen 621 greift in eine zweite Nutkurve an der Mantelfläche der zweiten Substanzträgerscheibe 3 ein. Während die Kurvenführungszapfen 611, 621 ins Innere des von dem Betätigungselement 6 gebildeten Kreissegments weisen, weist ein Verbindungselement 63 von dem Kreissegment nach außen. Es greift von Innen in eine hierzu vorgesehene Aufnahme der Abdeckung 13 ein. Wenn ein Benutzer die Abdeckung 13 um die gemeinsame Drehachse dreht, so bewegt er dabei auch die beiden Kurvenführungszapfen 611, 621 um die gemeinsame Drehachse. Diese Bewegung wird auf die beiden Substanzträgerscheiben 2, 3 übertragen.

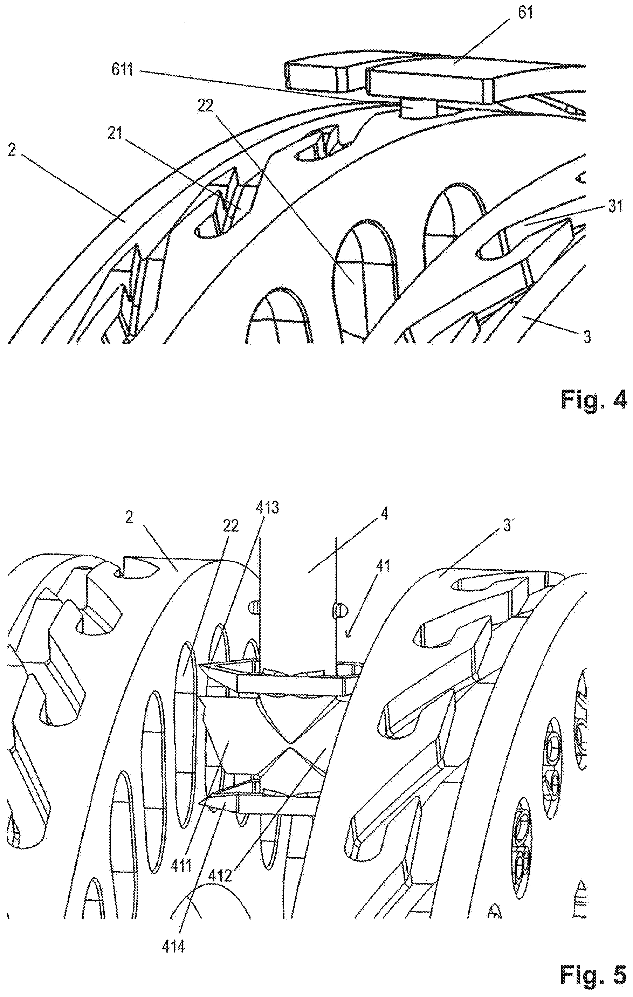

Fig. 4 zeigt die erste Nutkurve 21 der ersten Substanzträgerscheibe 2 und die zweite Nutkurve 31 der zweiten Substanzträgerscheibe 3. Weiterhin ist dargestellt, wie der erste Kurvenführungszapfen 611 in die erste Nutkurve 21 eingreift. Die Nuten der ersten Nutkurve 21 sind so geformt, dass der erste Kurvenführungszapfen 611 in einer Ruheposition in einer durch einen Hinterschnitt gebildeten Nut der ersten Nutkurve 21 ruht. Wird der erste Kurvenführungszapfen 611 nun entlang der Drehachse bewegt, so gleitet er entlang einer Schräge in der ersten Nutkurve 21 und drückt die erste Substanzträgerscheibe 2 dadurch entlang der Längsachse zu der Anstechdüse 41 hin. Nachdem diese Schräge überwunden wurde, wird der Kurvenführungszapfen in die nächste Nut der Nutkurve weitergeführt. Sobald er in diese eingreift, kehrt die erste Substanzträgerscheibe 2 entlang der Drehachse in ihre Ausgangsposition zurück. Lässt der Benutzer nun die Abdeckung 13 los, so federt das Betätigungselement 6 in seine Ausgangsposition zurück, übt dabei in Rotationsrichtung der ersten Substanzträgerscheibe 2 Druck gegen die Nut aus und dreht die erste Substanzträgerscheibe 2 dadurch um eine Nut weiter. Die erste Nutkurve 21 ist so ausgeführt, dass pro Kavität in der ersten Substanzträgerscheibe 2 jeweils eine Nut vorgesehen ist. Durch die Drehbewegung wird die erste Substanzträgerscheibe 2 deshalb um eine Kavität weitergedreht. Eine symmetrische Bewegung entlang der Drehachse und eine symmetrische Drehung in dieselbe Drehrichtung wird gleichzeitig durch den zweiten Kurvenführungszapfen 621 in Wechselwirkung mit der zweiten Nutkurve 31 an der zweiten Substanzträgerscheibe 3 ausgelöst.

Die Verbindung zwischen Abdeckung 13 und Betätigungselement 6 erfolgt dabei so, dass die Abdeckung 13 in der Ruheposition des Betätigungselements 6 das Mundstück 5 abdeckt und es so vor Verunreinigungen schützt.

Fig. 5 zeigt, wie die Anstechdüse 41 relativ zu den beiden Substanzträgerscheiben 2, 3 angeordnet ist. Die Anstechdüse 41 weist zwei Hohldüsen 411, 412 auf, die scharfkantig ausgeführt sind und orthogonal auf dem Luftkanal 4 stehen. Die erste Hohldüse 411 ist einer Kavität 22 der ersten Substanzträgerscheibe 2 zugewandt. Die zweite Hohldüse 412 ist einer in der Ansicht in Fig. 5 nicht erkennbaren Kavität der zweiten Substanzträgerscheibe 3 zugewandt. Oberhalb der ersten Hohldüse weist ein erstes Anstechelement 413 zu der Kavität 22 der ersten Substanzträgerscheibe 2 und unterhalb der ersten Hohldüse weist ein zweites Anstechelement 414 zu der Kavität 22 der ersten Substanzträgerscheibe 2. Auch oberhalb und unterhalb der zweiten Hohldüse ist jeweils an Anstechelement angeordnet, das jeweils der nicht erkennbaren Kavität der zweiten Substanzträgerscheibe 3 zugewandt ist.

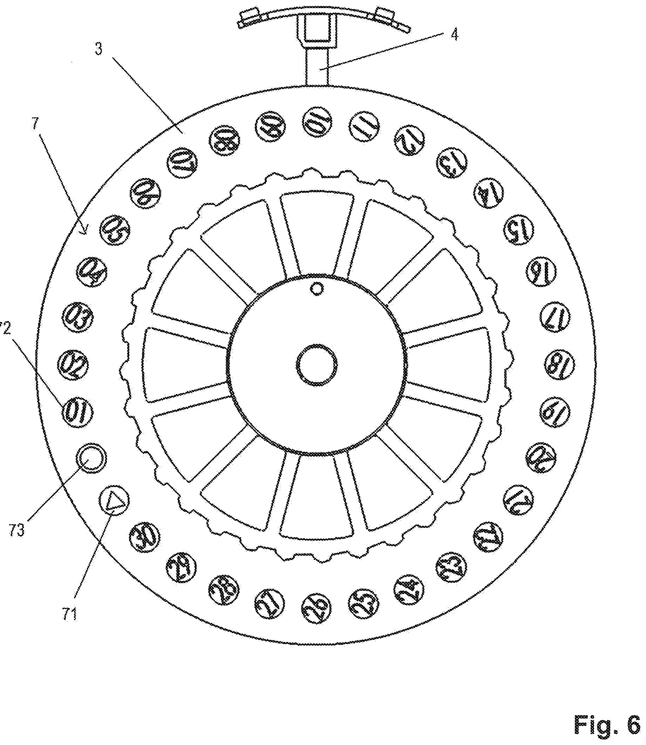

Wie in Fig. 6 dargestellt ist, weist die zweite Substanzträgerscheibe 3 an ihrer Außenseite, die ihren Kavitäten abgewandt ist, eine Kavitätenkennzeichnung 7 auf. Diese beginnt mit einer Startkavitätenkennzeichnung 71, die eine erste leere Kavität kennzeichnet. Die erste leere Kavität kann als Testkavität genutzt werden, um nach der Herstellung des Inhalators bei einer ersten Betätigung des Betätigungselements 6 dessen Funktionstüchtigkeit zu testen. Dann folgen von 30 bis 1 nummerierte Wirkstoffkavitätenkennzeichnungen 72 für Kavitäten, die jeweils einen Wirkstoff in Form einer pulverförmigen Trockensubstanz enthalten. Auf die letzte mit der Nr. 1 gekennzeichnete Wirkstoffkavitätenkennzeichnung 72 folgt eine Endkavitätenkennzeichnung 73. Diese kennzeichnet eine ebenfalls leere letzte Kavität der zweiten Substanzträgerscheibe 3. Die zweite Nutkurve 31 weist zwischen der Kavität mit der Startkavitätenkennzeichnung 71 und der Kavität mit der Endkavitätenkennzeichnung 73 eine Unterbrechung auf, sodass ein Weiterdrehen der zweiten Substanzträgerscheibe 3 nicht möglich ist. Durch eine Öffnung in der zweiten Gehäusehälfte 12 kann ein Benutzer des Inhalators die Wirkstoffkavitätenkennzeichnung der jeweils aktuellen Kavität erkennen.

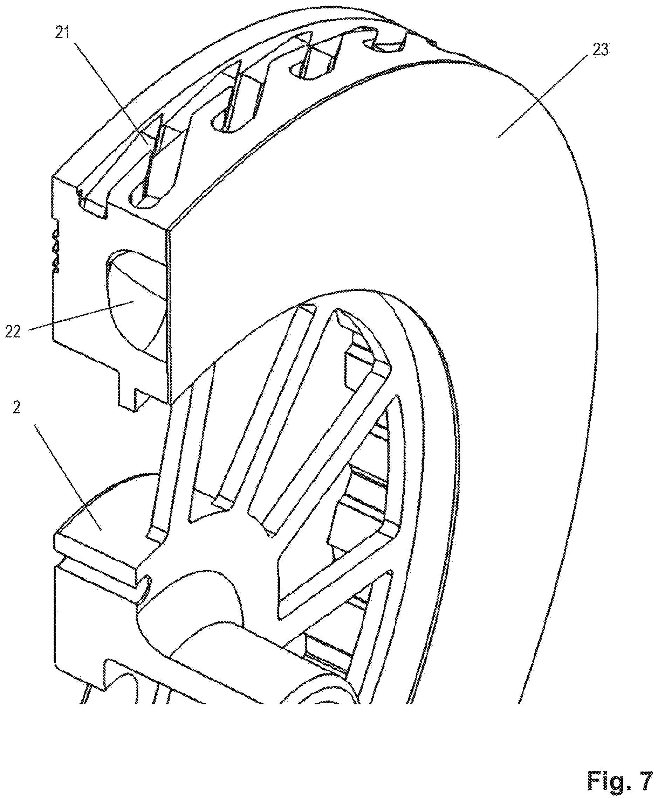

Die Kavitäten sind in den Fig. 1, 4 und 5 zum besseren Verständnis ohne Abdeckung dargestellt. Tatsächlich weisen sie jedoch jeweils eine Deckfolie auf. In Fig. 7 ist die Deckfolie 23 der Kavitäten 22 der ersten Substanzträgerscheibe dargestellt. Es handelt sich hierbei um eine Aluminiumfolie. Diese sorgt dafür, dass die pulverförmigen Trockensubstanzen im Inneren der Kavitäten 22 luftdicht abgeschlossen sind. Wenn die beiden Substanzträgerscheiben 2, 3 auf die Anstechdüse 41 zubewegt werden, durchschneiden die beiden Hohldüsen 411, 412 mit ihren scharfen Kanten die Deckfolie 23 der ersten Substanzträgerscheibe sowie die Deckfolie der zweiten Substanzträgerscheibe und eröffnen damit eine Verbindung zwischen den jeweiligen Kavitäten und der Anstechdüse 41. Außerdem schneiden die Anstechelemente 413, 141 weitere Öffnungen in die Deckfolie und ermöglichen so ein Einströmen von Luft in die Kavitäten. Dies ermöglicht es einem Benutzer, der durch das Mundstück 5 einatmet, die pulverförmigen Trockensubstanzen aus den Kavitäten durch die Anstechdüse 41, den Luftkanal 4 und die Auslassdüse 42 zu inhalieren, wobei diese fein zerstäubt werden. Luft strömt jeweils oberhalb und unterhalb der Hohldüsen 411, 412 in die Kavitäten, so dass die Kavitäten gleichmäßig und vollständig entleert werden können.

### Bezugszeichenliste

- 11:: erste Gehäusehälfte
- 12:: zweite Gehäusehälfte
- 13:: Abdeckung
- 14:: erster Deckel
- 15:: zweiter Deckel
- 2:: erste Substanzträgerscheibe
- 21:: erste Nutkurve
- 22:: Kavität
- 23:: Deckfolie
- 3:: zweite Substanzträgerscheibe
- 31:: zweite Nutkurve
- 4:: Luftkanal
- 41:: Anstechdüse
- 411:: erste Hohldüse
- 412:: zweite Hohldüse
- 413:: erstes Anstechelement
- 414:: zweites Anstechelement
- 42:: Auslassdüse
- 5:: Mundstück
- 6:: Betätigungselement
- 61:: erstes federndes Ende
- 611:: erster Kurvenführungszapfen
- 62:: zweites federndes Ende
- 621:: zweiter Kurvenführungszapfen
- 63:: Verbindungselement
- 7:: Kavitätenkennzeichnung
- 71:: Startkavitätenkennzeichnung
- 72:: Testkavitätenkennzeichnung
- 73:: Wirkstoffkavitätenkennzeichnung

## Patentansprüche

1. Inhalator zur Verabreichung pulverförmiger Trockensubstanzen, aufweisend zwei Substanzträgerscheiben (2, 3), die jeweils mit einer pulverförmigen Trockensubstanz befüllte Kavitäten (22) aufweisen, wobei die Substanzträgerscheiben (2, 3) so um eine gemeinsame Drehachse drehbar angeordnet sind, dass sich die Kavitäten (22) jeweils gegenüberliegen,
wobei der Inhalator einen Luftkanal (4) aufweist, der eine Anstechdüse (41) aufweist, **gekennzeichnet dadurch, dass** die Anstechdüse zwischen den Substanzträgerscheiben (2, 3) angeordnet ist, und dass die Anstechdüse (41) zwei Hohldüsen (411, 412) aufweist, wobei jede Hohldüse (411, 412) einer der Substanzträgerscheiben (2, 3) zugewandt ist, so dass die eine Hohldüse (411) eine Kavität (22) der einen Substanzträgerscheibe (2) anstechen kann, während die andere Hohldüse (412) eine Kavität (22) der anderen Substanzträgerscheibe (3) ansticht und die Trockensubstanzen aus den beiden Kavitäten (22) durch die beiden Hohldüsen (411, 412) transportierbar, in der Anstechdüse (41) mischbar und durch den Luftkanal (4) weitertransportierbar ist.

2. Inhalator nach Anspruch 1,
**dadurch gekennzeichnet, dass** jede Substanzträgerscheibe (2, 3) an ihrer Mantelfläche jeweils eine Nutkurve (21, 31) aufweist in welche jeweils ein Kurvenführungszapfen (611, 621) eingreift.

3. Inhalator nach Anspruch 2,
**dadurch gekennzeichnet, dass** die Nutkurven (21, 31) und die Kurvenführungszapfen (611, 621) eingerichtet sind, um eine lineare Verschiebung der Substanzträgerscheiben (2, 3) entlang der Drehachse und eine Drehbewegung der Substanzträgerscheiben (2, 3) zu bewirken.

4. Inhalator nach Anspruch 2 oder 3,
**dadurch gekennzeichnet, dass** die Nutkurven (21, 31) eingerichtet sind, um eine Anzahl von Drehungen ihrer Substanzträgerscheiben (2, 3) zu begrenzen.

5. Inhalator nach einem der Ansprüche 2 bis 4,
**dadurch gekennzeichnet, dass** er ein Betätigungselement (6) aufweist, welches die Kurvenführungszapfen (611, 621) aufweist.

6. Inhalator nach Anspruch 5,
**dadurch gekennzeichnet, dass** jeder Kurvenführungszapfen (611, 621) an einem federnden Ende (61, 62) des Betätigungselements (6) angeordnet ist.

7. Inhalator nach Anspruch 5 oder 6,
**dadurch gekennzeichnet, dass** die Kurvenführungszapfen (611, 621) so an dem Betätigungselement (6) angeordnet sind, dass sie bei Betätigung des Betätigungselements (6) eine synchrone Bewegung der beiden Substanzträgerscheiben (2, 3) bewirken.

8. Inhalator nach einem der Ansprüche 1 bis 7,
**dadurch gekennzeichnet, dass** die in den Substanzträgerscheiben (2, 3) angeordneten Kavitäten (22) jeweils mit einer Deckfolie (23) versiegelt sind.

9. Inhalator nach einem der Ansprüche 1 bis 8,
**dadurch gekennzeichnet, dass** die Anstechdüse (41) mehrere Anstechelemente (413, 414) aufweist, wobei jeder Substanzträgerscheibe (2, 3) jeweils eine Hohldüse (411, 412) und mehrere Anstechelemente (413, 414) zugewandt sind.

10. Inhalator nach einem der Ansprüche 1 bis 9,
**dadurch gekennzeichnet, dass** der Luftkanal (4) eine Auslassdüse (42) aufweist, die mit einem Mundstück (5) des Inhalators verbunden ist.

## Claims

1. Inhaler for administering powdery dry substances, having two substance carrier discs (2, 3), which each have cavities (22) filled with a powdery dry substance, wherein the substance carrier discs (2, 3) are arranged rotatably about a common axis of rotation such that the cavities (22) lie opposite each other,
wherein the inhaler has an air duct (4), which has a piercing nozzle (41), **characterized in that** the piercing nozzle is arranged between the substance carrier discs (2, 3), and **in that** the piercing nozzle (41) has two hollow nozzles (411, 412), wherein each hollow nozzle (411, 412) faces one of the substance carrier discs (2, 3), so that one hollow nozzle (411) can pierce a cavity (22) of the one substance carrier disc (2) while the other hollow nozzle (412) pierces a cavity (22) of the other substance carrier disc (3) and the dry substances can be transported from the two cavities (22) through the two hollow nozzles (411, 412), can be mixed in the piercing nozzle (41) and can be transported further through the air duct (4).

2. Inhaler according to Claim 1,
**characterized in that** each substance carrier disc (2, 3) has, on its circumferential surface, a cam groove (21, 31) in which a respective cam guide pin (611, 621) engages.

3. Inhaler according to Claim 2,
**characterized in that** the cam grooves (21, 31) and the cam guide pins (611, 621) are configured to cause a linear displacement of the substance carrier discs (2, 3) along the axis of rotation and a rotational movement of the substance carrier discs (2, 3).

4. Inhaler according to Claim 2 or 3,
**characterized in that** the cam grooves (21, 31) are configured to limit a number of rotations of their substance carrier discs (2, 3).

5. Inhaler according to any one of Claims 2 to 4,
**characterized in that** it has an actuation element (6), which has the cam guide pins (611, 621).

6. Inhaler according to Claim 5,
**characterized in that** each cam guide pin (611, 621) is arranged at a springloaded end (61, 62) of the actuation element (6).

7. Inhaler according to Claim 5 or 6,
**characterized in that** the cam guide pins (611, 621) are arranged on the actuation element (6) in such a way that they cause a synchronous movement of the two substance carrier discs (2, 3) when the actuation element (6) is actuated.

8. Inhaler according to any one of Claims 1 to 7,
**characterized in that** the cavities (22) arranged in the substance carrier discs (2, 3) are each sealed with a cover film (23).

9. Inhaler according to any one of Claims 1 to 8,
**characterized in that** the piercing nozzle (41) has a plurality of piercing elements (413, 414), wherein a hollow nozzle (411, 412) and a plurality of piercing elements (413, 414) face each substance carrier disc (2, 3).

10. Inhaler according to any one of Claims 1 to 9,
**characterized in that** the air duct (4) has an outlet nozzle (42), which is connected to a mouthpiece (5) of the inhaler.

## Revendications

1. Inhalateur pour l'administration de substances sèches en poudre, présentant deux disques de support de substance (2, 3) qui présentent chacun des cavités (22) remplies d'une substance sèche en poudre, les disques de support de substance (2, 3) étant agencés de manière à pouvoir tourner autour d'un axe de rotation commun de telle sorte que les cavités (22) se font respectivement face, l'inhalateur présentant un canal d'air (4) qui présente une buse de perçage (41), **caractérisé en ce que** la buse de perçage est agencée entre les disques de support de substance (2, 3), et **en ce que** la buse de perçage (41) présente deux buses creuses (411, 412), chaque buse creuse (411, 412) étant tournée vers l'un des disques de support de substance (2, 3), de telle sorte qu'une buse creuse (411) peut percer une cavité (22) d'un disque de support de substance (2), tandis que l'autre buse creuse (412) perce une cavité (22) de l'autre disque de support de substance (3), et les substances sèches peuvent être transportées à partir des deux cavités (22) à travers les deux buses creuses (411, 412), être mélangées dans la buse de perçage (41) et être transportées plus loin à travers le canal d'air (4).

2. Inhalateur selon la revendication 1,
**caractérisé en ce que** chaque disque de support de substance (2, 3) présente sur sa surface d'enveloppe respectivement une came à rainure (21, 31) dans laquelle s'engage respectivement un tenon de guidage de came (611, 621).

3. Inhalateur selon la revendication 2,
**caractérisé en ce que** les cames à rainure (21, 31) et les tenons de guidage de came (611, 621) sont adaptés pour effectuer un déplacement linéaire des disques de support de substance (2, 3) le long de l'axe de rotation et un mouvement de rotation des disques de support de substance (2, 3).

4. Inhalateur selon la revendication 2 ou 3,
**caractérisé en ce que** les cames à rainure (21, 31) sont adaptées pour limiter un nombre de rotations de leurs disques de support de substance (2, 3).

5. Inhalateur selon l'une quelconque des revendications 2 à 4,
**caractérisé en ce qu'**il présente un élément d'actionnement (6) qui présente les tenons de guidage de came (611, 621).

6. Inhalateur selon la revendication 5,
**caractérisé en ce que** chaque tenon de guidage de came (611, 621) est agencé à une extrémité élastique (61, 62) de l'élément d'actionnement (6).

7. Inhalateur selon la revendication 5 ou 6,
**caractérisé en ce que** les tenons de guidage de came (611, 621) sont agencés sur l'élément d'actionnement (6) de telle sorte que, lorsque l'élément d'actionnement (6) est actionné, ils provoquent un mouvement synchrone des deux disques de support de substance (2, 3).

8. Inhalateur selon l'une quelconque des revendications 1 à 7,
**caractérisé en ce que** les cavités (22) agencées dans les disques de support de substance (2, 3) sont chacune scellées par une feuille de couverture (23).

9. Inhalateur selon l'une quelconque des revendications 1 à 8,
**caractérisé en ce que** la buse de perçage (41) présente plusieurs éléments de perçage (413, 414), une buse creuse (411, 412) et plusieurs éléments de perçage (413, 414) étant respectivement tournés vers chaque disque de support de substance (2, 3).

10. Inhalateur selon l'une quelconque des revendications 1 à 9,
**caractérisé en ce que** le canal d'air (4) présente une buse de sortie (42) reliée à un embout buccal (5) de l'inhalateur.
